# EUROPÄISCHE PATENTSCHRIFT

(11) **EP 1 868 988 B1**
(45) Veröffentlichungstag und Bekanntmachung des Hinweises auf die Patenterteilung: **08.10.2008**
(21) Anmeldenummer: 06743262.5
(22) Anmeldetag: 07.04.2006
(51) Int. Cl.: C07C 253/30, C07C 255/03

(54) **VERFAHREN ZUR HERSTELLUNG VON GESÄTTIGTEN NITRILEN**
METHOD FOR PRODUCING SATURATED NITRILES
PROCEDE DE PRODUCTION DE NITRILES SATURES

(30) Priorität: 08.04.2005 DE 102005016489
(43) Veröffentlichungstag der Anmeldung: 26.12.2007
(73) Patentinhaber: BASF SE, 67056 Ludwigshafen (DE)
(72) Erfinder: EBERHARDT, Jan, 68167 Mannheim (DE); KRAUSE, Wolfgang, 68782 Brühl-rohrhof (DE)
(86) Internationale Anmeldenummer: PCT/EP2006/061431
(87) Internationale Veröffentlichungsnummer: WO 2006/106141

(56) Entgegenhaltungen:
- WO-A-20/04089879
- PATENT ABSTRACTS OF JAPAN Bd. 2003, Nr. 12, 5. Dezember 2003 (2003-12-05) & JP 2003 246769 A (UBE IND LTD), 2. September 2003 (2003-09-02)

## Beschreibung

### Technisches Gebiet der Erfindung:

Die vorliegende Erfindung betrifft ein Verfahren zur Herstellung von gesättigten Nitrilen durch katalytische Hydrierung der entsprechenden olefinisch ungesättigten Verbindungen in Gegenwart eines geträgerten Übergangsmetallkatalysators bei einem Druck von 1 bis 95 bar. Speziell betrifft die Erfindung ein Verfahren zur Herstellung von Tetrahydrogeranonitril (3,7-Dimethyloctannitril) durch katalytische Hydrierung von Geranonitril.

Tetrahydrogeranonitril (3,7-Dimethyloctannitril) ist ein Zwischenprodukt in der industriellen organischen Synthese. Es wird als Riechstoff oder Additiv in Kosmetika und Detergentien verwendet. Insbesondere bei der Verwendung als Riechstoff sind besondere Anforderungen an die Reinheit des Stoffes zu stellen. So beeinträchtigen bereits geringe Mengen von Verunreinigungen, speziell von Aminen, den Geruchseindruck des Materials erheblich. Bei der Verwendung in Kosmetika sollte darüber hinaus darauf geachtet werden, dass das einzusetzendeTetrahydrogeranonitril möglichst wenig olefinisch ungesättigte Nebenkomponenten bzw. Verunreinigungen aufweist, um Sensibilisierungsreaktionen vorzubeugen.

Als Herstellungsverfahren für gesättigte Nitrilverbindungen durch chemoselektive Hydrierung der C-C-Doppelbindungen sind Verfahren wie die Hydrierung von Geranylnitril unter Anwesenheit von modifiziertem Raney-Nickel bekannt wie von O.O.Volkova et al. in Maslozhirovaya Promyshlennnost, 1983, 4, 26-27 beschrieben. Bei dem Verfahren wird Geranonitril als Ausgangsstoff verwendet. Es wird die ungesättigte Bindung in α,β-Stellung hydriert. Je nach Versuchsbedingungen wird die Nitrilgruppe gleichzeitig zum Amin reduziert, so dass dieses Verfahren eine verminderte Ausbeute an Dihydrogeranonitril aufweist. Tetrahydrogeranonitril wird nicht gebildet. Daneben beschreiben die Autoren auch die Hydrierung von Geranonitril in Anwesenheit eines auf Aktivkohle geträgerten Palladiumkatalysators (5 % Pd/C) bei einem Druck von 100 bar und einer Temperatur von 30°C. Die Umsetzung führt zu einem Gemisch aus Tetrahydrogeranonitril (5 %) und dem Dimeren Bis(tetrahydrogeranyl)amin (19 %).

J. A. Profitt et al. beschreiben in J. Org. Chem. 1975, 40, 127 die Hydrierung von Geranylnitril in Gegenwart von Methanol, Magnesium sowie Salzsäure. Das Verfahren ermöglicht die selektive Hydrierung der α,β-ständigen C-C-Doppelbindung und liefert somit Citronellylnitril.

Die WO 2004/089879 offenbart ein Verfahren zur Herstellung von gesättigten oder ungesättigten Nitrilen, die zwei bis vier Isopren-Einheiten aufweisen. Die genannten Verbindungen werden durch selektive Hydrierung der zur Nitrilfunktion α,β-ständigen C-C-Doppelbindung und unter Erhalt eventueller weiterer C-C-Doppelbindungen aus den entsprechenden Vorläuferverbindungen erhalten. Die Hydrierung wird in Gegenwart von Aminverbindungen in einer Menge von 0,01 bis 100 Gew.-% (bezogen auf das umzusetzende Nitril) und in Gegenwart eines Palladiumkatalysators durchgeführt.

### Aufgabe der Erfindung:

Der vorliegenden Erfindung lag die Aufgabe zu Grunde, ein Verfahren zur Herstellung von gesättigten Nitrilen, speziell Tetrahydrogeranonitril, durch katalytische Hydrierung der entsprechenden ungesättigten Nitrile, speziell Geranonitril, bereitzustellen, bei dem auf den Zusatz von Aminen verzichtet werden kann und bei dem möglichst geringe Mengen von Aminen und/oder olefinisch ungesättigten Verbindungen als Nebenprodukte gebildet werden.

### Beschreibung Erfindung sowie der bevorzugten Ausführungsformen:

Es wurde ein Verfahren gefunden zur Herstellung von gesättigten Nitrilen der Formel (I) 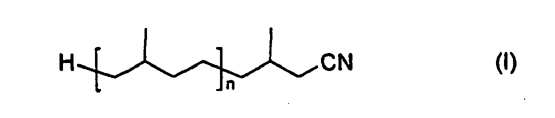 wobei der Index n eine ganze Zahl von 1 bis 3 bedeutet,
durch Umsetzung von ungesättigten Nitrilen der Formel (II) 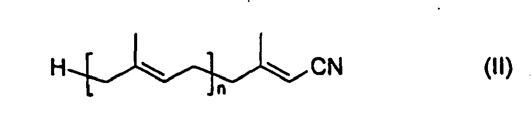 oder von ungesättigten Nitrilen der Formel (III) 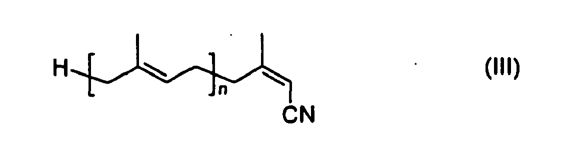 wobei der Index n jeweils eine ganze Zahl von 1 bis 3 bedeutet,
oder durch Umsetzung von Gemischen der ungesättigten Nitrile der Formeln (II) und (III) in Gegenwart von Wasserstoff und eines geträgerten Übergangsmetallkatalysators bei einem Druck von 1 bis 95 bar.

Als Ausgangsverbindungen zur Durchführung des erfindungsgemäßen Verfahrens eignen sich die bezüglich der zur Nitrilfunktion α,β-ständigen C-C-Doppelbindung isomeren Verbindungen der Formeln (II) und (III), die jeweils als solche oder in Form eines Gemisches untereinander eingesetzt werden können. Die Ausgangsverbindungen der Formeln (II) bzw. (III), bei denen der Index n 2 oder 3 bedeutet, können jeweils auch in Form von Isomerengemischen bezüglich der nicht α,β-ständigen C-C-Doppelbindung bzw. -Doppelbindungen eingesetzt werden.

Darüber hinaus eignen sich als Ausgangsverbindungen auch solche, die in Form von Gemischen mit Nebenkomponenten vorliegen, wobei die Nebenkomponenten insbesondere solche Verbindungen sein können, die durch Hydrierung von einer oder mehreren C-C-Doppelbindungen aus den Verbindungen der Formeln (II) oder (III) erhalten werden können oder die Isomere bezüglich der Lage der C-C-Doppelbindungen der genannten Verbindungen darstellen. Beispielhaft seien für die Umsetzung von Geranylnitril oder Nerylnitril zu Tetrahydrogeranonitril als mögliche Nebenkomponenten genannt: Citronellylnitril und Isomere des Geranonitrils wie 3,7-Dimethyl-2,7-octadiennitril, 3,7-Dimethyl-3,6-octadiennitril. Die jeweiligen Nebenkomponenten können in Mengen von jeweils etwa 0,1 bis etwa 25 Gew.-% (bezogen auf die Gesamtmenge an umzusetzendem Gemisch) in dem umzusetzendem ungesättigten Nitril enthalten sein.

Der Index n in den Formeln (I), (II) und (III) bedeutet eine ganze Zahl von 1 bis 3. Im Rahmen einer bevorzugten Ausführungsform des erfindungsgemäßen Verfahrens bedeutet n die Zahl 1 entsprechend einem Verfahren zur Herstellung von Tetrahydrogeranonitril der Formel (IV) 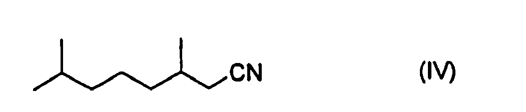 durch Umsetzung von Geranylnitril der Formel (V) 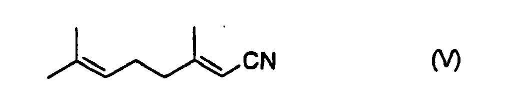 oder von Nerylnitril der Formel (VI) 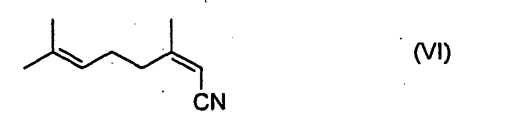 oder Gemischen von Geranylnitril und Nerylnitril.

Ein im Rahmen der vorliegenden Erfindung insbesondere bevorzugter Ausgangstoff ist Geranonitril, das ein Gemisch darstellt, das zu etwa gleichen Teilen aus Geranylnitril der Formel (V) und Nerylnitril der Formel (VI) besteht.

Die erfindungsgemäße Umsetzung wird in Gegenwart von Wasserstoff durchgeführt. Der Wasserstoff kann in reiner Form oder in Form von Gemischen mit anderen, vorzugsweise inerten Gasen wie beispielsweise Argon und/oder Stickstoff eingesetzt werden. Bevorzugt führt man die Reaktion unter Wasserstoffatmosphäre durch.

Die erfindungsgemäße Umsetzung wird darüber hinaus in Gegenwart eines geträgerten Übergangsmetallkatalysators durchgeführt. Erfindungsgemäß bevorzugte Übergangsmetallkatalysatoren sind Suspensionskatalysatoren, insbesondere solche, die als Aktivkomponenten eines oder mehrere Metalle ausgewählt aus der Gruppe der Metalle Pd, Ag, Ru, Rh, Ni bevorzugt Pd enthalten. Als Trägermaterialien der erfindungsgemäß einzusetzenden Katalysatoren kommen beispielsweise Aktivkohle, Aluminiumoxid, Kieselgel, CaCO₃, BaSO₄, ZrO₂, TiO₂, bevorzugt Aktivkohle und Alumiumoxid und besonders bevorzugt Aktivkohle in Betracht.

Ein im Rahmen der vorliegenden Erfindung besonders bevorzugter Katalysator ist Pd auf Aktivkohle. Die genannten Katalysatoren weisen vorteilhafterweise Metallgehalte von etwa 0,1 bis etwa 20 Gew.-%, bevorzugt von etwa 0,5 bis etwa 15 Gew.-% und besonders bevorzugt von etwa 4 bis etwa 11 Gew.-% (jeweils bezogen auf das reduzierte Metall des fertigen Katalysators) auf. Derartige Katalysatoren sind kommerziell zugänglich und beispielsweise unter den Bezeichnungen Degussa E1002, Degussa E101, Degussa E105, Degussa E106, Engelhard C3630, Heraeus K201, Heraeus K202, Heraeus K203, Heraeus K204, Heraeus K219 erhältlich.

Der gewählte Katalysator wird vorteilhaft in einer solchen Menge eingesetzt, dass der Gehalt an Übergangsmetall, bevorzugt Pd, bezogen auf die Menge an jeweils eingesetztem ungesättigten Nitril, beispielsweise bezogen auf die Menge an umzusetzenden Geranonitril, etwa 0,00001 bis etwa 1,0 Gew.-%, bevorzugt etwa 0,0001 bis etwa 0,1 Gew.-% und besonders bevorzugt von etwa 0,001 bis etwa 0,05 Gew.-% beträgt.

Das Verfahren wird erfindungsgemäß bei einem absoluten Druck im Bereich von 1 bis 95 bar durchgeführt. Bevorzugt führt man das Verfahren bei einem Druck im Bereich von etwa 7 bis etwa 70 bar, besonders bevorzugt im Bereich von etwa 10 bis etwa 50 bar durch.

Vorteilhaft führt man das erfindungsgemäße Verfahren bei Temperaturen im Bereich von etwa 20°C bis etwa 200°C, bevorzugt im Bereich von etwa 35°C bis etwa 150°C und besonders bevorzugt im Bereich von etwa 50°C bis etwa 100°C durch.

Das erfindungsgemäße Verfahren wird üblicherweise in Suspensionsfahrweise in flüssiger Phase durchgeführt. Es kann in Gegenwart eines unter den Reaktionsbedingungen inerten Lösungsmittel das zur Durchführung von Hydrierungen geeignet ist wie beispielsweise, Dioxan, Tetrahydrofuran, Methanol, Isopropanol, Hexan oder ohne Lösungsmittel durchgeführt werden. Im Rahmen einer bevorzugten Ausführungsform, insbesondere zur Herstellung von Tetrahydrogeranonitril arbeitet man ohne Zusatz von Lösungsmittel.

Das erfindungsgemäße Verfahren kann auch in einer gemischten Flüssig-/Gasphase durchgeführt werden, wobei sich bevorzugt mehr als 50 Gew.-% des Fluids, in der Flüssigphase befinden.

Der erfindungsgemäß einzusetzende Trägerkatalysator kann mit gutem Erfolg in Form von handelsüblichen, Wasser enthaltenden Darreichungsformen eingesetzt werden. Im Rahmen einer weiteren bevorzugten Ausführungsform des erfindungsgemäßen Verfahrens setzt man die gewählten Trägerkatalysatoren in einer Form ein, die durch einen möglichst geringen Wassergehalt gekennzeichnet ist. Bevorzugt setzt man solche Katalysatoren, bevorzugt einen auf Aktivkohle oder Aluminiumoxid geträgerten Pd-Katalysator, ein, die einen Wassergehalt von 0 bis etwa 70 Gew.-%, besonders bevorzugt von etwa 0,1 bis etwa 60 Gew.-%, ganz besonders bevorzugt von etwa 0,1 bis etwa 50 Gew.-%, insbesondere bevorzugt von etwa 0,5 bis etwa 30 Gew.-%, noch mehr bevorzugt von etwa 0,5 bis etwa 10 Gew.-% und am meisten bevorzugt von etwa 0,5 bis etwa 5 Gew.-% aufweisen. Derartige Katalysatoren sind kommerziell zugänglich oder können durch dem Fachmann an sich bekannte Verfahren, beispielsweise durch Vortrocknen aus solchen zugänglich gemacht werden.

Das erfindungsgemäße Verfahren kann mit gutem Erfolg ohne Zusatz von Promotoren oder Hilfsstoffen durchgeführt werden. Dabei sind unter Promotoren oder Hilfsstoffen im Rahmen der vorliegenden Erfindung beispielsweise Aminverbindungen, speziell aliphatische oder aromatische organische Amine wie beispielsweise Trimethylamin, Triethylamin, Tri-n-butylamin, Tri-n-octylamin, Diethylamin, Diisopropylamin, Di-n-butylamin, Di-sec-butylamin, n-Butylamin, sec-Butylamin, tert-Butylamin, n-Octylamin, Triethanolamin, Diethanolamin, Ethanolamin, N,N,N',N'-Tetramethylethylendiamin, 3-Methoxypropylamin, 3-Ethoxypropylamin, 3-(Methylamino)propylamin, 3-(Dimethylamino)propylamin, 3-(Dibutylamino)propylamin, Morpholin, 1,4-Diazabicyclo[2.2.2]-octan, 1,5-Diazabicyclo[4.3.0]nonen-5 sowie 1,8-Diazabicyclo[5.4.0]undecen-7, Pyridin, N,N-Dimethylaminopyridin sowie Chinolin zu verstehen. Unter Promotoren sind auch Dotierungen des jeweils eingesetzten Katalysators zu verstehen, wobei als Dotierungsmetalle beispielsweise Zink, Cadmium, Silber, Kupfer und Mangan genannt seien. Demzufolge eignen sich die vorstehend genannten, kommerziell erhältlichen Katalysatoren ohne weitere Modifizierungen oder Zusatzstoffe zum Einsatz im Rahmen des erfindungsgemäßen Verfahrens.

Erfindungsgemäß bevorzugt sind Verfahren zur Herstellung von Tetrahydrogeranonitril der Formel (IV) durch katalytische Hydrierung von Geranylnitril, Nerylnitril oder Gemischen derselben oder bevorzugt von Geranonitril in Gegenwart eines geträgerten Palladiumkatalysators, wobei die Hydrierung ohne Zusatz von Aminverbindungen durchgeführt wird. Die Umsetzung wird im Rahmen einer wiederum bevorzugten Ausführungsform bei einer Temperatur von etwa 20°C bis 200°C und einem Druck von etwa 1 bis etwa 95 bar, besonders bevorzugt bei einer Temperatur von etwa 35°C bis etwa 150°C und einem Druck von etwa 7 bis etwa 70 bar durchgeführt und am meisten bevorzugt bei einer Temperatur von etwa 50°C bis etwa 100°C und einem Druck von etwa 10 bis etwa 50 bar durchgeführt.

Zur Durchführung des erfindungsgemäßen Verfahrens eigenen sich die dem Fachmann bekannten und zur Durchführung von Hydrierungen in Suspensionsfahrweise geeignet erscheinenden Reaktoren wie beispielsweise Rührautoklaven oder gepackte Blasensäulen.

Das erfindungsgemäße Verfahren kann diskontinuierlich halb- oder vollkontinuierlich durchgeführt werden.

Die erfindungsgemäße Umsetzung ist, in Abhängigkeit von den gewählten Reaktionsbedingungen, üblicherweise nach etwa 1 bis etwa 24 h, oft nach etwa 12 h weitgehend abgeschlossen. Das rohe Reaktionsgemisch wird in üblicher Weise von dem eingesetzten Katalysator getrennt, vom gegebenenfalls eingesetzten Lösungsmittel befreit und gewünschtenfalls durch geeignete Verfahren, beispielsweise durch Destillation, weiter aufgereinigt bzw. getrennt.

Das erfindungsgemäße Verfahren eröffnet einen wirtschaftlich leistungsfähigen und verfahrenstechnisch vorteilhaften Zugang zu gesättigten Nitrilen, insbesondere zu Tetrahydrogeranonitril. Letzteres zeichnet sich durch geringen Gehalt aminischer Nebenkomponenten bzw. Verunreinigungen aus, wodurch sich eine weitere Aufreinigung zur Herstellung von Tetrahydrogeranonitril in Riechstoffqualität oft erübrigt.

### Beispiele:

Die folgenden Beispiele dienen der Veranschaulichung der Erfindung, ohne sie in irgendeiner Weise zu beschränken:

Die Versuche zu den Beispielen 1 bis 16 sowie dem Vergleichsbeispiel wurden im Labor in einem 300 ml Rührautoklaven durchgeführt, die Versuche in den Beispielen 17 und 18 wurden in einem Technikumsautoklav mit Volumen 9 Liter durchgeführt. Geranonitril und der jeweilige Katalysator wurden im Autoklaven vorgelegt. Der Autoklav wurde mit Stickstoff gespült und Wasserstoff wurde aufgepresst. Anschließend wurde das Reaktionsgemisch auf Reaktionstemperatur erhitzt. Die Rührerdrehzahl betrug im Laborversuch 1000 U/min, im Technikumsversuch (Beispiele 17 und 18) betrug die Drehzahl 600 U/min.

Die Reaktionsausträge wurden mittels Gaschromatographie analysiert (Trennsäule DBWAX, Länge 30 m; Innendurchmesser 0,32 mm; Trägergas Stickstoff; Temperaturprogramm: 80°C, danach Erwärmung um 3°C/min auf 230°C, abschließend 10 min isotherm bei 230°C). Umsätze, Ausbeuten und Selektivitäten werden im Folgenden auf Basis von GC-Flächenprozenten angegeben.

### Beispiel 1:

100 g technisches Geranonitril (Gehalt 97,7 %) und 2 g eines Katalysators aus 5 Gew.-% Pd auf Aktivkohle mit einem Wassergehalt von 53 Gew.-% (entspr. 0,05 Gew.-% Pd bezüglich Geranonitril) wurden vorgelegt. Die Hydrierung wurde bei einem Wasserstoffdruck von 15 bis 29 bar und einer Reaktionstemperatur von 25 bis 31 °C durchgeführt. Nach einer Reaktionszeit von 18 h wurde ein Geranonitril-Umsatz von 95,1 % bei einer Produktselektivität bezüglich Tetrahydrogeranonitril von 75,4 % erreicht: Als überwiegende Nebenkomponente wurde teilhydriertes Geranylamin (Dihydrogeranylamin) in einer Menge von 14,9 % gefunden.

### Beispiel 2:

100 g technisches Geranonitril (Gehalt 97,7 %) und 1 g eines Katalysator aus 5 Gew.-% Pd auf Aktivkohle mit einem Wassergehalt von 3 Gew.-% (entspr. 0,05 Gew.-% Pd bezüglich Geranonitril) wurden vorgelegt. Die Hydrierung wurde bei einem Wasserstoffdruck von 12 bis 28 bar und einer Reaktionstemperatur von anfangs 25°C durchgeführt. Die Temperatur erhöhte sich im Verlauf der Reaktion auf bis zu 63°C. Nach einer Reaktionszeit von 315 min wurde ein Geranonitril-Umsatz von 99,7 % bei einer Produktselektivität bezüglich Tetrahydrogeranonitril von 99,8 % erreicht.

### Beispiel 3:

100 g technisches Geranonitril (Gehalt 97,7 %) und 0,5 g eines Katalysators aus 5 Gew.-% Pd auf Aktivkohle mit einem Wassergehalt von 3 Gew.-% (entspr. 0,025 Gew.-% Pd bezüglich Geranonitril) wurden vorgelegt. Die Hydrierung wurde bei einem Wasserstoffdruck von 8 bis 29 bar und einer Reaktionstemperatur von anfangs 50°C durchgeführt. Die Temperatur erhöhte sich im Verlauf der Reaktion auf bis zu 69°C. Nach einer Reaktionszeit von 220 min wurde die Heizung abgestellt. Die Reaktionsmischung wurde weitere 14 h bei 20 bar Wasserstoffdruck gerührt. Anschließend wurde der Autoklav entspannt. Der Geranonitril-Umsatz betrug 98,4 %, die Selektivität bezüglich Tetrahydrogeranonitril betrug 99,6 %.

### Beispiel 4:

100 g technisches Geranonitril (Gehalt 97,7 %) und 0,1 g eines Katalysators aus 5 Gew.-% Pd auf Aktivkohle mit einem Wassergehalt von 3 Gew.-% (entspr. 0,005 Gew.-% Pd bezüglich Geranonitril) wurden vorgelegt. Die Hydrierung wurde bei einem Wasserstoffdruck von 9 bis 31 bar und einer Reaktionstemperatur von 60°C durchgeführt. Nach einer Reaktionszeit von 330 min wurde die Heizung abgestellt. Die Reaktionsmischung wurde weitere 14 h bei 29 bar Wasserstoffdruck gerührt. Ein Geranonitril-Umsatz von 69,7 % wurde ermittelt. Die Selektivität bezüglich Tetrahydrogeranonitril betrug 83,5 %.

Der Reaktionsaustrag wurde weitere 240 Minuten bei einem Wasserstoffdruck von 11 bis 29 bar und einer Reaktionstemperatur von 100°C umgesetzt. Der Umsatz steigerte sich auf 95,4 % bei einer Selektivität bezüglich Tetrahydrogeranonitril von 97,1 %.

### Beispiel 5:

100 g technisches Geranonitril (Gehalt 97,7 %) und 1,0 g eines Katalysators aus 5 Gew.% Pd auf Aktivkohle mit einem Wassergehalt von 3 Gew.-% (entspr. 0,005 Gew-% Pd) wurden vorgelegt. Die Hydrierung wurde bei einem Wasserstoffdruck von 5 bis 30 bar und einer Reaktionstemperatur von 80°C durchgeführt. Nach einer Reaktionszeit von 240 min wurden im Reaktionsaustrag ein Gehalt von 99,7 % (GC-Flächenprozent) Tetrahydrogeranonitril ermittelt.

Der Reaktionsaustrag wurde aus dem Reaktor entnommen und zur Katalysatorabtrennung filtriert. Der Katalysator wurde mit Geranonitril in den Autoklav überführt und für weitere Geranonitril-Hydrierungen unter den gleichen Reaktionsbedingungen eingesetzt. Im zweiten Reaktionslauf wurde nach 240 min Reaktionszeit bei 80°C und 5 bis 30 bar Wasserstoffdruck ein Tetrahydrogeranonitril-Gehalt von wiederum 99,7 % erreicht. Im dritten Reaktionslauf wurde nach 4-stündiger Reaktion bei gleichen Bedingungen ein Tetrahydrogeranonitril-Gehalt von 99,4 % erzielt.

### Beispiel 6:

100 g technisches Geranonitril (Gehalt 97,7 %) und 1,0 g eines Katalysators aus 10 Gew.-% Pd auf Aktivkohle mit einem Wassergehalt von etwa 5 Gew.-% (Degussa E101 N/D; entspr. 0,01 Gew.-% Pd bezüglich Geranonitril) wurden vorgelegt. Die Hydrierung wurde bei einem Wasserstoffdruck von 6 bis 29 bar und einer Reaktionstemperatur von anfangs 50°C durchgeführt. Die Temperatur erhöhte sich im Verlauf der Reaktion auf bis zu 70°C. Nach einer Reaktionszeit von 240 min wurde die Heizung abgestellt. Die Reaktionsmischung wurde weitere 14 h bei 26 bar Wasserstoffdruck gerührt. Anschließend wurde die Reaktionsmischung bei 60°C und 25 bar Wasserstoffdruck weitere 240 min gerührt. Der Geranonitril-Umsatz betrug 99,1 %, die Selektivität bezüglich Tetrahydrogeranonitril betrug 99,7 %.

### Beispiel 7:

100 g technisches Geranonitril (Gehalt 97,7 %) und 1,0 g eines Katalysators aus 5 Gew.-% Pd auf Aktivkohle mit einem Wassergehalt von 3 Gew.-% (entspr. 0,05 Gew.-% Pd bezüglich Geranonitril) wurden vorgelegt. Die Hydrierung wurde bei einem Wasserstoffdruck von 4 bis 30 bar und einer Reaktionstemperatur von 150°C durchgeführt. Nach einer Reaktionszeit von 45 min wurde ein Geranonitril-Umsatz von 99,9 % ermittelt. Der Tetrahydrogeranonitril-Gehalt betrug 99,3 %.

### Beispiel 8:

150 g Geranonitril (Gehalt 98,9 %) und 1,5 g eines Katalysators aus 5 Gew.-% Pd auf Aktivkohle mit einem Wassergehalt von 3 Gew.-% (entspr. 0,05 Gew.-% Pd bezüglich Geranonitril) wurden vorgelegt. Die Hydrierung wurde bei einem Wasserstoffdruck von 30 bar und einer Reaktionstemperatur von 75°C durchgeführt. Nach einer Reaktionszeit von 330 min wurde ein Geranonitril-Umsatz von über 99,9 % ermittelt. Der Tetrahydrogeranonitril-Gehalt des Produkts betrug 96,4 %. Als überwiegende Nebenkomponente wurde das tertiäre Amin Tris(3,7-dimethyloctyl)amin in einer Menge von 3,2 % nachgewiesen.

### Beispiel 9:

150 g Geranonitril (Gehalt 98,9 %) und 3,0 g eines Katalysators aus 5 Gew.-% Pd auf Aktivkohle mit einem Wassergehalt von 53 Gew.-% (entspr. 0,05 Gew.-% Pd bezüglich Geranonitril) wurden vorgelegt. Die Hydrierung wurde bei einem Wasserstoffdruck von 30 bar und einer Reaktionstemperatur von 80°C durchgeführt. Nach einer Reaktionszeit von 360 min wurde ein Geranonitril-Umsatz von über 99,9 % ermittelt. Der Tetrahydrogeranonitril-Gehalt des Produkts betrug 84,9 %. Der Gehalt am tertiären Amin Tris(3,7-dimethyloctyl)amin betrug 13,9 %.

### Beispiel 10:

150 g technisches Geranonitril (Gehalt 97,7 %) und 3,0 g eines Katalysators aus 5 Gew.-% Pd auf Aktivkohle mit einem Wassergehalt von 53 Gew.-% (entspr. 0,05 Gew.-% Pd bezüglich Geranonitril) wurden vorgelegt. Die Hydrierung wurde bei einem Wasserstoffdruck von 30 bar und einer Reaktionstemperatur von 75°C durchgeführt. Nach einer Reaktionszeit von 360 min wurde ein Geranonitril-Umsatz von über 99,9 % ermittelt. Der Tetrahydrogeranonitril-Gehalt betrug 96,3 %. Als Nebenkomponente wurden 2,2% des tertiären Amins Tris(3,7-dimethyloctyl)amin nachgewiesen.

### Beispiel 11:

150 g Geranonitril (Gehalt 98,9 %) und 3,0 g eines Katalysators aus 5 Gew.% Pd auf Aktivkohle mit einem Wassergehalt von 50 Gew.% (entspr. 0,05 Gew-% Pd bezüglich Geranonitril) wurden vorgelegt. Die Hydrierung wurde bei einem Wasserstoffdruck von 30 bar und einer Reaktionstemperatur von 75°C durchgeführt. Nach einer Reaktionszeit von 270 min wurde ein Geranonitril-Umsatz von über 99,9 % ermittelt. Der Tetrahydrogeranonitril-Gehalt betrug 84,7 %. Der Gehalt an tertiären Amin Tris(3,7-dimethyloctyl)amin betrug 14,1 %.

### Beispiel 12:

150 g technisches Geranonitril (Gehalt 97,7 %) und 3,0 g eines Katalysators aus 5 Gew.-% Pd auf Aktivkohle mit einem Wassergehalt von 53 Gew.-% (entspr. 0,05 Gew.-% Pd bezüglich Geranonitril) wurden vorgelegt. Die Hydrierung wurde bei einem Wasserstoffdruck von 5 bar und einer Reaktionstemperatur von 50°C durchgeführt. Nach einer Reaktionszeit von 320 min wurde ein Geranonitril-Umsatz von 99,4 % ermittelt. Der Tetrahydrogeranonitril-Gehalt im Reaktionsaustrag betrug 71,9 %, der Anteil an Tris(3,7-dimethyloctyl)amin betrug 0,3 %. Citronellylnitril wurde mit einem Gehalt von 19,8 % nachgewiesen.

### Beispiel 13:

150 g Geranonitril (Gehalt 98,9 %) und 0,1 g eines Katalysators aus 5 Gew.-% Pd auf Aktivkohle mit einem Wassergehalt von 53 Gew.-%, entspr. 0,0017 Gew.-% Pd bezüglich Geranonitril) wurden vorgelegt. Die Hydrierung wurde bei einem Wasserstoffdruck von 50 bar und einer Reaktionstemperatur von 60 bis 77°C durchgeführt. Nach einer Reaktionszeit von 290 min wurde ein Geranonitril-Umsatz von 99,0 % ermittelt. Der Tetrahydrogeranonitril-Gehalt im Reaktionsaustrag betrug 71,9 %, der Anteil an Tris(3,7-dimethyloctyl)amin betrug 0,2 %. Citronellylnitril wurde mit einem Gehalt von 25,0 % nachgewiesen.

### Beispiel 14:

150 g Geranonitril (Gehalt 98,9 %) und 1,5 g eines Katalysators aus 5 Gew.-% Pd auf Aktivkohle mit einem Wassergehalt von 53 Gew.-%, entspr. 0,025 Gew.-% Pd bezüglich Geranonitril) wurden vorgelegt. Die Hydrierung wurde bei einem Wasserstoffdruck von 5 bar und einer Reaktionstemperatur von 70 bis 81 °C durchgeführt. Nach einer Reaktionszeit von 360 min wurde ein Geranonitril-Umsatz von über 99,9 % ermittelt. Der Tetrahydrogeranonitril-Gehalt im Reaktionsaustrag betrug 84,5 %, der Anteil an Tris(3,7-dimethyloctyl)amin betrug 0,6 %. Citronellylnitril wurde mit einem Gehalt von 13,7 % nachgewiesen.

### Beispiel 15:

100 g Geranonitril (Gehalt 98,9 %) und 0,2 g eines Katalysators aus 5 Gew.-% Pd auf Aktivkohle mit einem Wassergehalt von 53 Gew.-%, entspr. 0,005 Gew.-% Pd bezüglich Geranonitril) wurden vorgelegt. Die Hydrierung wurde bei einem Wasserstoffdruck von 20 bar und einer Reaktionstemperatur von 144 bis 162°C durchgeführt. Nach einer Reaktionszeit von 240 min wurde ein Geranonitril-Umsatz von über 99,9 % ermittelt. Der Tetrahydrogeranonitril-Gehalt im Reaktionsaustrag betrug 98,1 %, der Anteil an Tris(3,7-dimethyloctyl)amin betrug 0,8 %.

### Beispiel 16:

150 g Geranonitril (Gehalt 98,9 %) und 1,5 g eines Katalysators aus 5 Gew.-% Pd auf Aktivkohle mit einem Wassergehalt von 53 Gew.% (entspr. 0,025 Gew.-% Pd bezüglich Geranonitril) wurden vorgelegt. Die Hydrierung wurde bei einem Wasserstoffdruck von 70 bar und einer Reaktionstemperatur von 85 bis 102°C durchgeführt. Nach einer. Reaktionszeit von 240 min wurde ein Geranonitril-Umsatz von 99,7 % ermittelt. Der Tetrahydrogeranonitril-Gehalt im Reaktionsaustrag betrug 96,5 %, der Anteil an Tris(3,7-dimethyloctyl)amin betrug 2,4 %.

### Beispiel 17:

5200 g technisches Geranonitril (Gehalt 97,7 %) und 31,2 g eines Katalysators aus 5 Gew.-% Pd auf Aktivkohle mit einem Wassergehalt von ca. 50 Gew.-% (entspr. 0,015 Gew.-% Pd bezüglich Geranonitril) wurden vorgelegt. Die Hydrierung wurde bei einem Wasserstoffdruck von 10 bar und einer Reaktionstemperatur von 60°C begonnen. Nach 60 Minuten Reaktionszeit wurde die Reaktionstemperatur alle 60 Minuten in Schritten von 5°C auf 80°C erhöht. Nach Erreichen der Reaktionstemperatur von 80°C wurde der Reaktionsdruck innerhalb von 2 h auf 20 bar erhöht. Nach weiteren 5 Stunden Reaktionszeit wurde der Wasserstoffdruck innerhalb von 1 h auf 30 bar erhöht. Nach weiteren 5 Stunden Reaktionszeit wurde die Reaktionstemperatur innerhalb einer Stunde von 80 auf 100°C erhöht. Abschließend wurde die Reaktion weitere 5 Stunden bei 100 °C und 30 bar gefahren.

Nach einer Reaktionszeit von insgesamt 16 h wurde ein Geranonitril-Umsatz von über 99,9 % bei einer Produktselektivität bezüglich Tetrahydrogeranonitril von 99,8 % erreicht. Als einzige Nebenkomponente wurde Tris(3,7-dimethyloctyl)amin in einer Menge von 0,2 % nachgewiesen. Die Produktzusammensetzung änderte sich in der verbleibenden Reaktionszeit nicht.

### Beispiel 18:

5200 g technisches Geranonitril (Gehalt 97,7 %) und der aus Beispiel 17 rückgewonnene Katalysator wurden vorgelegt. Die Hydrierung wurde bei den gleichen Reaktionsbedingungen wie in Beispiel 17 durchgeführt. Die Aktivität des wiedereingesetzten Katalysators war geringfügig schlechter als in Beispiel 17. Nach einer Reaktionszeit von 16 h wurde ein Geranonitril-Umsatz von über 99,9 % ermittelt, der Tetrahydrogeranonitril-Gehalt betrug 96,8 %, der Anteil an Dihydrogeranonitril betrug 3,0 %. Nach einer Reaktionszeit von insgesamt 21 h wurde ein Geranonitril-Umsatz von über 99,9 % bei einer Produktselektivität bezüglich Tetrahydrogeranonitril von 99,8 % erreicht. Als Nebenkomponente wurde Tris(3,7-dimethyloctyl)amin in einer Menge von 0,2 % nachgewiesen.

### Vergleichsbeispiel:

150 g Geranonitril (Gehalt 98,9 %) und 4,5 g Raney-Nickel wurden vorgelegt. Die Hydrierung wurde bei einem Wasserstoffdruck von 10 bis 15 bar und einer Reaktionstemperatur von 40°C durchgeführt. Nach einer Reaktionszeit von 330 min wurde ein Geranonitril-Umsatz von 19,2 % ermittelt. Der Tetrahydrogeranonitril-Gehalt betrug 0,3 %. Der Gehalt an Dihydrogeranonitril (Citronellylnitril) betrug 16,4 %.

Die Reaktion wurde weitere 390 min bei einem Wasserstoffdruck von 30 bar und einer Reaktionstemperatur von 50°C fortgesetzt. Der Geranonitril-Umsatz verbesserte sich auf 48,9 %. Der Tetrahydrogeranonitril-Gehalt im Reaktionsaustrag betrug 0,5 %, der Gehalt an Dihydrogeranonitril betrug 43,2 %.

## Patentansprüche

1. Verfahren zur Herstellung von gesättigten Nitrilen der Formel (I) 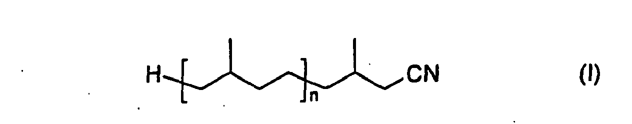 wobei der Index n eine ganze Zahl von 1 bis 3 bedeutet,
durch Umsetzung von ungesättigten Nitrilen der Formel (II) 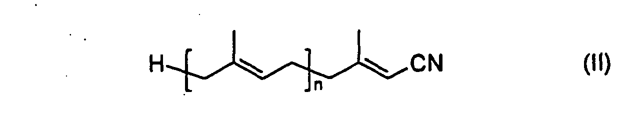 oder von ungesättigten Nitrilen der Formel (III) 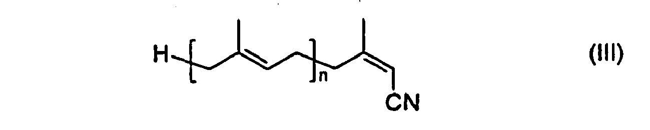 wobei der Index n jeweils eine Ganze Zahl von 1 bis 3 bedeutet,
oder durch Umsetzung von Gemischen der ungesättigten Nitrile der Formeln (II) und (III) in Gegenwart von Wasserstoff und eines geträgerten Übergangsinetallkatalysators bei einem Druck von 1 bis 95 bar.

2. Verfahren nach Anspruch 1 zur Herstellung von Tetrahydrogeranonitril der Formel (IV) 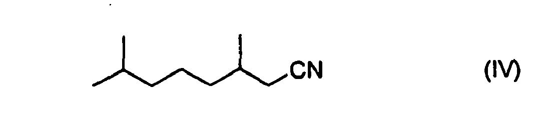 durch Umsetzung von Geranylnitril der Formel (V) 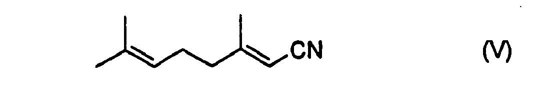 oder von Nerylnitril der Formel (VI) 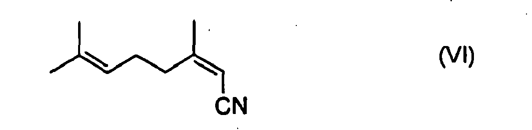 oder Gemischen von Geranylnitril und Nerylnitril.

3. Verfahren nach einem der Ansprüche 1 oder 2, **dadurch gekennzeichnet, dass** man die Umsetzung bei einem Druck von 7 bis 70 bar durchführt.

4. Verfahren nach einem der Ansprüche 1 bis 3, **dadurch gekennzeichnet, dass** man die Umsetzung bei einem Druck von 10 bis 50 bar durchführt.

5. Verfahren nach einem der Ansprüche 1 bis 4, **dadurch gekennzeichnet, dass** man die Umsetzung bei einer Temperatur von 20°C bis 200°C durchführt.

6. Verfahren nach einem der Ansprüche 1 bis 5, **dadurch gekennzeichnet, dass** man die Umsetzung bei einer Temperatur von 50°C bis 100°C durchführt.

7. Verfahren nach einem der Ansprüche 1 bis 6, **dadurch gekennzeichnet, dass** der geträgerte Übergangsmetallkatalysator als Aktivkomponente Pd enthält.

8. Verfahren nach einem der Ansprüche 1 bis 7, **dadurch gekennzeichnet, dass** der geträgerte Übergangsmetallkatalysator Aktivkohle als Träger aufweist.

9. Verfahren nach einem der Ansprüche 1 bis 8, **dadurch gekennzeichnet, dass** der geträgerte Übergangsmetallkatalysator Aluminiumoxid als Träger aufweist.

10. Verfahren nach einem der Ansprüche 1 bis 9, **dadurch gekennzeichnet, dass** man einen auf Aktivkohle geträgerten Pd-Katalysator einsetzt.

11. Verfahren nach Anspruch 10, **dadurch gekennzeichnet, dass** der Katalysator einen Wassergehalt von bis zu 10 Gew.-% aufweist.

12. Verfahren nach einem der Ansprüche 1 bis 11, **dadurch gekennzeichnet, dass** man den Katalysator in einer solchen Menge einsetzt, dass der Gehalt an Übergangsmetall bezogen auf die Menge an umzusetzendem Nitril 0,00001 bis 1,0 Gew.-% beträgt.

13. Verfahren nach einem der Ansprüche 1 bis 12, **dadurch gekennzeichnet**, **dadurch gekennzeichnet, dass** man den Katalysator in einer solchen Menge einsetzt, dass der Gehalt an Übergangsmetall bezogen auf die Menge an umzusetzendem Nitril 0,001 bis 0,05 Gew.-% beträgt.

14. Verfahren nach einem der Ansprüche 1 bis 13, **dadurch gekennzeichnet, dass** man die Umsetzung ohne Zusatz von Promotoren oder Hilfsstoffen durchführt.

15. Verfahren nach einem der Ansprüche 1 bis 14, **dadurch gekennzeichnet, dass** man die Umsetzung ohne Zusatz von Aminverbindungen durchführt.

16. Verfahren nach einem der Ansprüche 2 bis 15, **dadurch gekennzeichnet, dass** man Geranylnitril oder Nerylnitril oder Gemische von Geranylnitril und Nerylnitril in Form von Gemischen mit einer oder mehreren Nebenkomponenten einsetzt, wobei die Nebenkomponenten ausgewählt sind aus der Gruppe Citronellylnitril, 3,7-Dimethyl-2,7-octadiennitril und 3,7-Dimethyl-3,6-octadiennitril und in einer auf die Gesamtmenge an umzusetzendem Gemisch bezogenen Menge von jeweils 0,1 bis 25 Gew.-% enthalten sind.

## Claims

1. A process for the preparation of saturated nitriles of the formula (I) 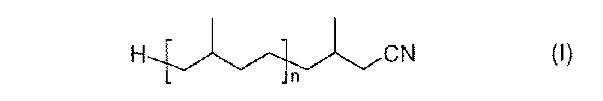 where the index n is an integer from 1 to 3,
by converting unsaturated nitriles of the formula (II) 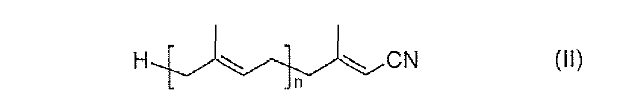 or unsaturated nitriles of the formula (III) 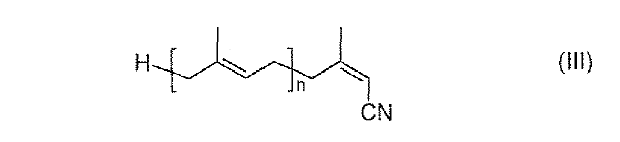 where the index n is in each case an integer from 1 to 3,
or by converting mixtures of the unsaturated nitriles of the formulae (II) and (III) in the presence of hydrogen and a supported transition metal catalyst at a pressure of from 1 to 95 bar.

2. The process according to claim 1 for the preparation of tetrahydrogeranonitrile of the formula (IV) 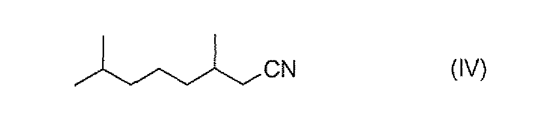 by converting geranylnitrile of the formula (V) 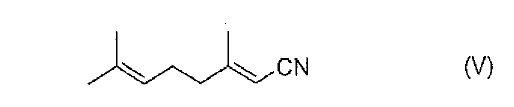 or nerylnitrile of the formula (VI) 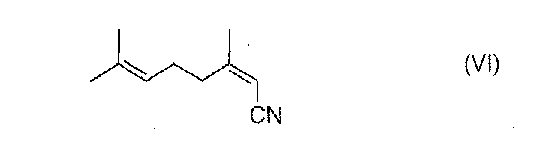 or mixtures of geranylnitrile and neryinitrile.

3. The process according to either of claims 1 and 2, wherein the conversion is carried out at a pressure of from 7 to 70 bar.

4. The process according to one of claims 1 to 3, wherein the conversion is carried out at a pressure of from 10 to 50 bar.

5. The process according to one of claims 1 to 4, wherein the conversion is carried out at a temperature of from 20°C to 200°C.

6. The process according to one of claims 1 to 5, wherein the conversion is carried out at a temperature of from 50°C to 100°C.

7. The process according to one of claims 1 to 6, wherein the supported transition metal catalyst comprises Pd as active component.

8. The process according to one of claims 1 to 7, wherein the supported transition metal catalyst has activated carbon as the support.

9. The process according to one of claims 1 to 8, wherein the supported transition metal catalyst has aluminum oxide as the support.

10. The process according to one of claims 1 to 9, wherein a Pd catalyst supported on activated carbon is used.

11. The process according to claim 10, wherein the catalyst has a water content of up to 10% by weight.

12. The process according to one of claims 1 to 11, wherein the catalyst is used in an amount such that the content of transition metal, based on the amount of nitrile to be converted, is 0.00001 to 1.0% by weight.

13. The process according to one of claims 1 to 12, wherein the catalyst is used in an amount such that the content of transition metal, based on the amount of nitrile to be converted, is 0.001 to 0.05% by weight.

14. The process according to one of claims 1 to 13, wherein the conversion is carried out without the addition of promoters or auxiliaries.

15. The process according to one of claims 1 to 14, wherein the conversion is carried out without the addition of amine compounds.

16. The process according to one of claims 2 to 15, wherein geranylnitrile or nerylnitrile or mixtures of geranylnitrile and neryinitrile are used in the form of mixtures with one or more secondary components, the secondary components being chosen from the group consisting of citronellylnitrile, 3,7-dimethyl-2,7-octadienenitrile and 3,7-dimethyl-3,6-octadienenitrile and are present in an amount, based on the total amount of mixture to be converted, of in each case 0.1 to 25% by weight.

## Revendications

1. Procédé de préparation de nitriles saturés de formule (I) 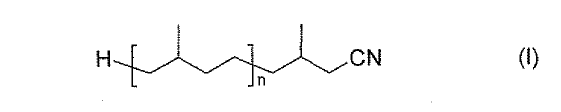 dans laquelle l'indice n représente un entier de 1 à 3,
par la réaction de nitriles insaturés de formule (II) 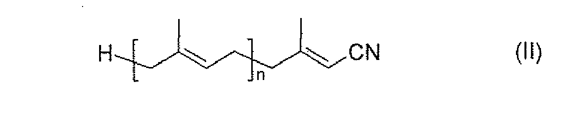 ou de nitriles insaturés de formule (III) 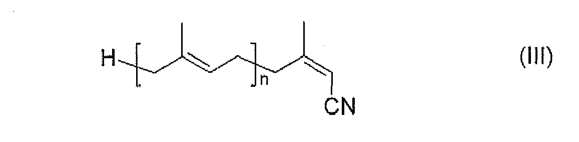 dans lesquelles l'indice n représente, dans chaque cas, un entier de 1 à 3,
ou par la réaction de mélanges des nitriles insaturés de formules (II) et (III) en présence d'hydrogène et d'un catalyseur à métal de transition sur support à une pression de 1 à 95 bars.

2. Procédé selon la revendication 1 pour la préparation du tétrahydrogéranonitrile de formule (IV) 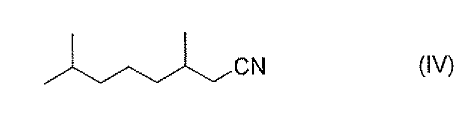 par la réaction du géranylnitrile de formule (V) 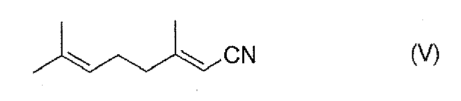 ou du nérylnitrile de formule (VI) 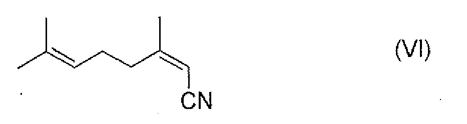 ou de mélanges de géranylnitrile et de nérylnitrile.

3. Procédé selon l'une quelconque des revendications 1 ou 2, **caractérisé en ce que** la réaction est effectuée à une pression de 7 à 70 bars.

4. Procédé selon l'une quelconque des revendications 1 à 3, **caractérisé en ce que** la réaction est effectuée à une pression de 10 à 50 bars.

5. Procédé selon l'une quelconque des revendications 1 à 4, **caractérisé en ce que** la réaction est effectuée à une température de 20 °C à 200 °C.

6. Procédé selon l'une quelconque des revendications 1 à 5, **caractérisé en ce que** la réaction est effectuée à une température de 50 °C à 100 °C.

7. Procédé selon l'une quelconque des revendications 1 à 6, **caractérisé en ce que** le catalyseur à métal de transition sur support contient du Pd en tant que composant actif.

8. Procédé selon l'une quelconque des revendications 1 à 7, **caractérisé en ce que** le catalyseur à métal de transition sur support contient du charbon actif en tant que support.

9. Procédé selon l'une quelconque des revendications 1 à 8, **caractérisé en ce que** le catalyseur à métal de transition sur support contient de l'oxyde d'aluminium en tant que support.

10. Procédé selon l'une quelconque des revendications 1 à 9, **caractérisé en ce que** l'on utilise un catalyseur au Pd sur support de charbon actif.

11. Procédé selon la revendication 10, **caractérisé en ce que** le catalyseur présente une teneur en eau allant jusqu'à 10 % en poids.

12. Procédé selon l'une quelconque des revendications 1 à 11, **caractérisé en ce que** l'on utilise le catalyseur en une quantité telle que la teneur en métal de transition, par rapport à la quantité de nitrile à réagir, est de 0,00001 à 1,0 % en poids.

13. Procédé selon l'une quelconque des revendications 1 à 12, **caractérisé en ce que** l'on utilise le catalyseur en une quantité telle que la teneur en métal de transition, par rapport à la quantité de nitrile à réagir, est de 0,001 à 0,05 % en poids.

14. Procédé selon l'une quelconque des revendications 1 à 13, **caractérisé en ce que** la réaction est effectuée sans l'addition de promoteurs ou d'auxiliaires.

15. Procédé selon l'une quelconque des revendications 1 à 14, **caractérisé en ce que** la réaction est effectuée sans l'addition de composés amine.

16. Procédé selon l'une quelconque des revendications 2 à 15, **caractérisé en ce que** l'on utilise du géranylnitrile ou du nérylnitrile ou des mélanges de géranylnitrile et de nérylnitrile sous la forme de mélanges avec un ou plusieurs composants secondaires, les composants secondaires étant choisis parmi le groupe constitué du citronellylnitrile, du 3,7-diméthyl-2,7-octadiène-nitrile et du 3,7-diméthyl-3,6-octadiènenitrile, et étant présents en une quantité, par rapport à la quantité totale du mélange à réagir, de 0,1 à 25 % en poids, dans chaque cas.
